# EUROPEAN PATENT SPECIFICATION

(11) **EP 1 813 223 B1**
(45) Date of publication and mention of the grant of the patent: **15.04.2009**
(21) Application number: 07250233.9
(22) Date of filing: 20.01.2007
(51) Int. Cl.: A61F 2/01, A61F 2/84

(54) **Stent delivery system to improve placement accuracy for self-expanding stent**
Stenteinführsystem zur Verbesserung der Positioniergenauigkeit für einen selbstexpandierbaren Stent
Système de mise en place d'endoprothèse vasculaire pour améliorer la précision d'une endoprothèse vasculaire auto-étendue

(30) Priority: 27.01.2006 US 343258
(43) Date of publication of application: 01.08.2007
(73) Proprietor: Cordis Corporation, Miami Lakes, FL 33014 (US)
(72) Inventor: Wang, Huisun, Maple Grove, MN 55311 (US)
(74) Representative: Belcher, Simon James

(56) References cited:
- WO-A2-20/04082530
- DE-A1- 19 936 207
- US-B1- 6 544 279

## Description

The present invention generally relates to medical devices, particularly a stent delivery system for a self-expanding stent that is provided with structure that prevents stent jumping during deployment.

A stent is a generally longitudinal tubular device formed of biocompatible material(s) that is useful in the treatment of stenoses, strictures or aneurysms in blood vessels and other body vessels. Stents can be implanted within an unhealthy vessel to reinforce collapsing, partially occluded, weakened, or abnormally dilated sections of the vessel. Typically, stents are employed after angioplasty of a blood vessel to prevent restenosis of the diseased vessel. While stents are most notably used in blood vessels, stents may also be implanted in other body vessels such as the urogenital tract and the bile duct. A stent may exhibit flexibility to allow it to be inserted through curved vessels. Furthermore, stents are often initially configured in a radially compressed state, such as by crimping, to facilitate delivery and deployment in intraluminal catheter implantation.

Stents are akin to scaffoldings in their support of the passageway. Structurally, a stent may have two or more struts or wire support members connected together into a lattice-like frame. As indicated, stents may be in a compressed state prior to delivery and deployment, as compression facilitates insertion through small cavities. Stents can be delivered to the desired implantation site percutaneously in a catheter or similar transluminal device. With a lattice-like structure, a portion of the stent surface area is open, such openings defined by the struts that form the stent. Open spaces are desirable in that they allow plaque from the lesion to fall through the stent and enter the blood stream.

Carotid artery stenting is becoming a more prevalent option in treating carotid artery diseases (stenosis). In carotid artery stenting procedure, a relatively small stent of about 8-10 mm diameter and about 20 mm long may be used. A stent may be configured as an elongate structure, generally cylindrical in shape. The stent may exist in a first, predeployed state. The stent can be transformed into a second state, post delivery, with the stent, in the second state, having a substantially greater diameter than the diameter of the stent in the first state. The stent can be implanted in the vessel of a patient using the stent delivery system appropriate for the type of stent being delivered to the vessel.

Certain kinds of stents are self expanding, and other kinds, such as the Palmaz-Schatz® stent, available from Cordis Corporation of Miami Lakes, FL, USA, are expanded radially outward by the force imparted by an inflated angioplasty type balloon as it pushes against the inner stent walls. An example of a self-expanding stent is the SMART® nitinol stent, a nickel titanium alloy stent also available from the Cordis Corporation.

Typically, a stent may be delivered in an introducer sheath. The stent and sheath can be advanced to a site within the patient's vessel through a guide catheter. A self-expanding stent possesses a spring force that causes the stent to expand following its implacement in the vessel when a restraining sheath is retracted from the compressed stent. Alternatively, and by way of example, a self-expanding nitinol stent may be of the kind that expands when warmed above the martensitic transition temperature for the nitinol alloy (e.g., above 30° C.)

Self-expanding stent delivery systems (SDS) are provided with an outer sheath into which the stent is loaded. The distal end of the outer sheath member is retracted toward the proximal end of the SDS in order to deploy a self-expanding stent. Because the outer sheath may develop slack, the inner member and tip of the stent Delivery System (SDS) tend to move forward when the outer member is pulled back.

Upon deployment, a self-expanding stent expands row by row as the surrounding outer sheath is retracted. In a successful deployment, the initially expanded rows of the stent should anchor to the vessel wall to set the position of the stent. Rows that expand subsequently will then expand outward and contact the vessel wall.

In a successful deployment the stent is positioned at the desired location within the vessel. However, self-expanding stents exhibit spring-like characteristics, so care must be taken to reduce, if not eliminate, the phenomenon of "stent jumping". That is, self-expanding stents can store energy. Frictional force generated as the outer sheath is retracted can cause the stent perform like a spring, storing energy as the frictional force acts on the stent. The stored energy is released as the stent expands beyond the end of the sheath, and this release of energy can cause the stent to move or "jump" from the desired position, resulting in inaccurate placement. Inaccurate stent placement could render stent deployment as ineffective in treating stenosis.

Accordingly, it is desirable to provide a device that minimizes, if not eliminates, stent jumping in order to provide for more accurate placement of self-expanding stents.

The improvement of stent placement accuracy is an object of the invention.

The minimization, if not the elimination, of stent jumping is a further object of the invention.

WO-A-2004/082530 recognises that gravity and torque acting on a delivery device for an expandable intraluminal device can orient the delivery device near a vessel wall. It discusses a number of devices for spacing an expandable intraluminal medical device from a vessel wall before delivery. In one embodiment a basket device expands when a sheath is axially retracted from the basket and at the same time forces a portion of an expandable intraluminal device against a vessel wall.

US-6544279 discusses a vascular filter. The filter comprises a hoop attached to a guidewire with a filter sax affixed to the hoop. The vascular filer can be collapsed to a small size, enabling it to be retracted into the guidewire lumen of stent delivery systems.

The present invention is directed to a stent delivery system for the delivery of a self-expanding stent that is provided with structure that facilitates accurate stent deployment at the desired location. This arrangement is intended to diminish stent jumping, if not eliminate it, which as indicated above adversely affects stent deployment.

According to an aspect of the present invention, there is provided a stent delivery system as defined in appended claim 1. According to another aspect of the present invention, there is provided a stent delivery system as defined in appended claim 2.

In one embodiment, the stent delivery system has an outer sheath forming an elongated tubular member having distal and proximal ends and an inside and outside diameter. The stent delivery system also includes an inner shaft located coaxially within the outer sheath. The inner shaft has a distal end, a proximal end and a longitudinal axis extending therebetween. The stent delivery system is configured to retain a self-expanding stent located within the outer sheath, wherein the stent makes frictional contact with the outer sheath and the shaft is disposed coaxially within a lumen of the stent. The SDS is further provided with structure that upon release and deployment improves stent placement accuracy and reduces, if not eliminates, stent jumping. In one aspect of the invention, the structure is positioned distal to the position on the delivery device where the stent is retained. For example the structure can be positioned intermediate the location where the stent is retained prior to its deployment and the distal end of the delivery device. Alternatively, the structure may be positioned along the guide wire at a location distal to the delivery device. Preferably, the structure may be an off-center support member that initially is retained in a non deployed state within a lumen, which for example could be the outer sheath of the SDS which also retains the stent, it may be the distal tip of the SDS. Further, regardless of where the off center support member may be located on the SDS, it is advantageous to engage same to the SDS in a non-coaxial manner. In other words, upon expansion, the axis of the off center support member is not coaxial with the inner shaft of the device, for reasons that will become apparent below.

In an exemplary arrangement, the off center support member is retained within the outer sheath and is mounted non-coaxially to the inner shaft. When the outer sheath is retracted in the stent deployment process, the off center support member, which in one inventive aspect is made of an expandable, spring like material, expands at an orientation that is not coaxial, relative to the longitudinal axis of the device. The off center support member expands to the interior vessel wall and makes contact with same. The vessel walls apply a counterforce to the interior shaft of the SDS, forcing the SDS into an off-center arrangement within the vessel. Now, with the SDS in the off center arrangement, and further sheath retraction, the self-expanding stent is exposed. This arrangement causes initial off-center deployment of the stent, which is believed to facilitate the anchoring of the stent to the walls of the vessel. Thus, the structural attributes of the present delivery system should reduce, if not eliminate, the stent jumping effect that is believed to result in inaccurate stent deployments.

In an alternative embodiment, the off center support member is a vascular device, such as a vascular filter or thrombectomy/embolectomy device, in which a blood permeable sac having a support loop forming a rim around the open end of the sac. The support hoop can be attached to the distal region of the stent delivery system or other elongated member, such as a guide wire. When deployed and open, the support hoop defines an opening in the sac.

Embodiments of the invention will now be described by way of example with reference to the accompanying drawings, in which:
Figure 1 is a simplified elevational view of a stent delivery system in that can be modified in accordance with the present invention;
Figure 2 depicts a cross sectional view through a vessel wall of an embodiment of a stent delivery system of the present invention;
Figure 3 depicts a cross sectional view through a vessel wall of another embodiment of a stent delivery system of the present invention;
Figure 4 is a cross sectional view of an aspect of the embodiment shown in Fig. 3.

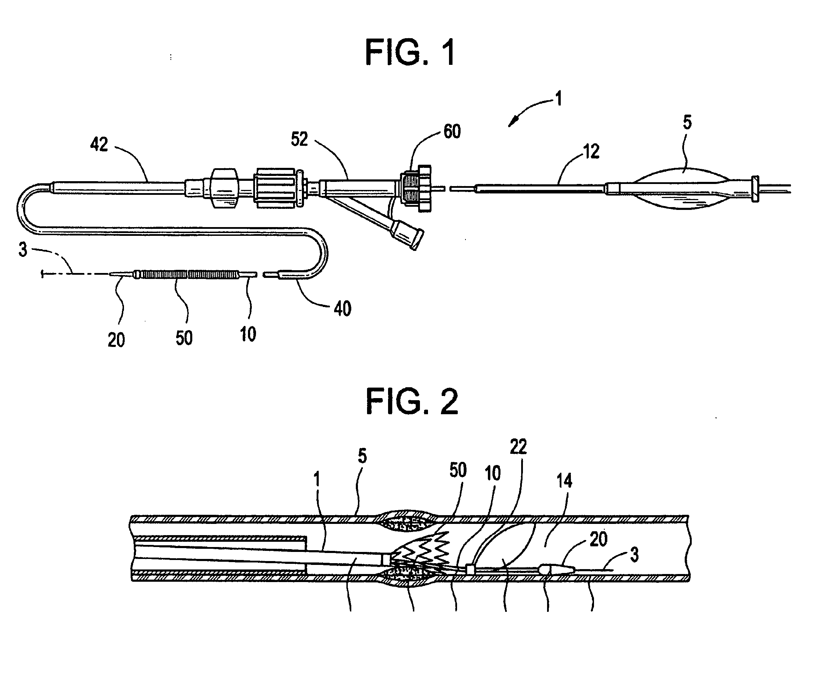

The structural attributes of the present invention, which facilitate an initial off center stent deployment, may be incorporated into existing stent delivery systems. For that reason, the features of the stent delivery systems disclosed in US-6,773,446 and US-6,939,352, each of which share the common assignee with the present application, are given as examples of stent delivery systems that can be modified to include the off centering structural attributes within the ambit of this disclosure. However, the present invention need not be bound to the specific features and embodiments of these particular patent disclosures. In any event, Figure 1 illustrates a conventional stent delivery system of the kind disclosed in US-6,773,446.

FIG. 2 shows a self-expanding stent delivery stent delivery system 1 made in accordance with the present invention in the course of deployment within a body vessel 5, such as a blood vessel. Stent delivery system 1 comprises inner and outer coaxial tubes, wherein the inner tube is identified as shaft 10 and the outer tube is identified as the sheath 40. A self-expanding stent 50 is located within the outer sheath, wherein (at least prior to deployment) the stent makes frictional contact with the outer sheath. As seen in Fig. 1, shaft 10 is disposed coaxially within a lumen of the stent.

At the distal end of the shaft, the shaft 10 has distal tip 20 attached thereto. Distal tip 20 can be made from any number of materials known in the art including polyamide, polyurethane, polytetraflouroethylene, and polyethylene, in multi-layer or single layer structures. The distal tip 20 has a near end 34 whose diameter can approximate that of the outer diameter of the sheath 40 adjacent to the distal tip. Furthermore the distal tip tapers to a smaller diameter from its proximal end 34 to its distal end 36. A guide wire 3 passing out of the delivery device is used to guide the device to the stent deployment site during navigation through the vessel passages. Specifically as shown, the distal tip 36 can be hollow to give the distal end of the tip the ability to slide over and therefore be directed by the guide wire 3.

Shaft 10 is provided with marker 22. In an aspect of the invention, the marker 22 is located distal to the stent bed 24, that is, the place where the stent is retained on the delivery device prior to its deployment. Marker 22 can be made from any number of materials, such as stainless steel, and is even more preferably made from a highly radio-opaque material such as platinum, gold, tantalum, or radio-opaque filled polymer. The marker can be attached to shaft 10 by mechanical or adhesive bonding, or by any other means known to those skilled in the art. Stent 50 resides coaxially over the stent bed 24 so that stent bed 24 is located within the lumen of stent 50.

An off center support member 100 is engaged to the marker 22. In relation to shaft 10, off center support member 100 is mounted to shaft 10 non-coaxially. Off center support member 100 can be mounted to shaft 10 by mechanical or adhesive bonding, or by any other means known to those skilled in the art. Off center support member can be formed of a self-expanding material, such as nitinol. At least when fully expanded, the off center support member should possess the size and dimensions necessary to contact the interior vessel wall and cause a counterforce to be applied against the shaft 10 of the SDS, forcing the SDS into an off-center arrangement within the vessel. Merely by way of example, the off center support member 100 can be in the shape of a circular ring, an oval, or an ellipse. The off center support member need not be closed, as for example it may be horseshoeshaped. Again, the off center support member can be in any configuration, but preferably is not coaxial relative to the longitudinal axis of the shaft 10, and preferably possesses size and dimensions that will displace the shaft off center when the off center support member is deployed and in contact with the interior vessel wall.

Off center support member 100 is preferably made from a superelastic alloy such as Nitinol. Most preferably, off center support member 100 is made from an alloy comprising from about 50.5% (as used herein these percentages refer to atomic percentages) Ni to about 60% Ni, and most preferably about 55% Ni, with the remainder of the alloy Ti. Off center support member 100 can be nitinol wire, although it may also be formed from a multi-strand nitinol cable, or a spring tempered stainless steel, or other super-elastic material.

In a ready-to-use state, the distal end of sheath retains the inner shaft 10, stent 50 upon stent bed 24, the marker 22, and the off center support member 100. The sheath may extend out to the distal tip 36.

In operation, the stent delivery system 1 is inserted into a vessel and advanced along guide wire 3 so that the stent bed 24 moves to the target deployment site. Once the physician determines, by known techniques, that the marker 22 on shaft 10 is where it should be in relation to the targeted disease site, the physician initiates retraction of the sheath in order to deploy the device. This is accomplished by operating the controls on the stent delivery system. As the outer sheath retracts, off center support member 100, distal to where the stent is retained on the stent bed 24, deploys first. As the off center support member is preferably made of a self expanding material, it expands as it is released from the constraints of the sheath. Furthermore, as the off center support member is mounted to the shaft 10 in a non-coaxial arrangement, it expands non-coaxially relative to the longitudinal axis of the shaft 10. The off center support member expands to and contacts the interior vessel wall 5, applying a counterforce to the shaft 10 of the stent delivery system, forcing the stent delivery system into an off-center arrangement within the vessel 5. Thus, as shown in Fig 2, the stent delivery system is displaced off center. As further shown in Fig. 2, the self-expanding stent is exposed as the physician continues to retract the sheath. Thus, the initial rows of the stent are deployed at an angled arrangement believed to anchor more efficiently to the vessel walls than in the case where the inner shaft is not in an off center arrangement.

By initially deploying the self-expanding stent in an off center arrangement, the leading row of struts possessed by the stent are angled towards the vessel walls, resulting in a relatively quick anchoring of the stent to the walls. As the sheath is retracted, and the compression energy of the compressed stent is released, the stent should be restrained from jumping due to the improved initial anchoring of the stent. This arrangement is believed to prevent, if not eliminate, stent jumping. Thus, the structural attributes of the present delivery system should reduce, if not eliminate, the stent jumping effect that is believed to result in inaccurate stent deployments.

After the sheath is fully retracted, and the stent fully expands and is engaged against the vessel walls, the stent deployment system, including the off center support member, is removed from the patient.

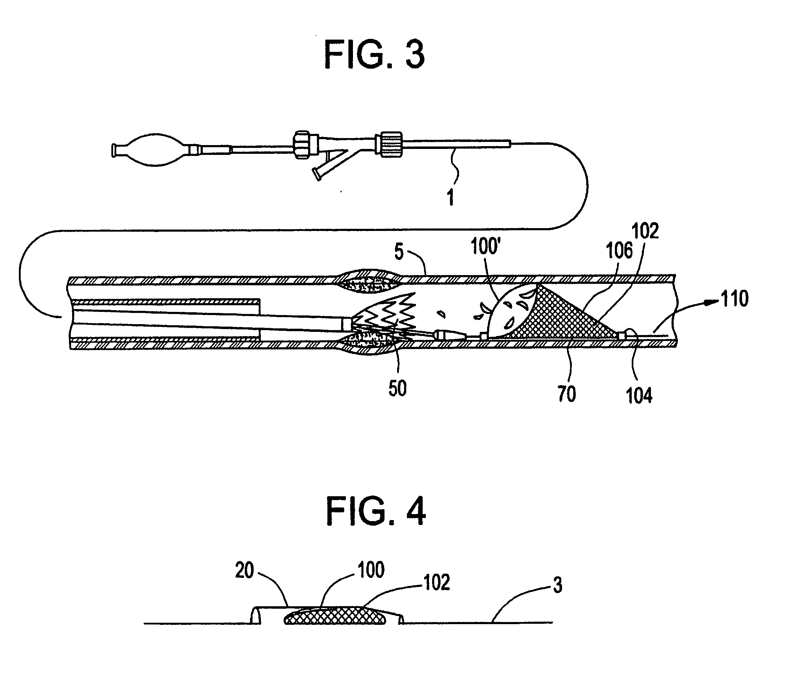

Fig. 3 depicts an alternative embodiment in which the off center support member is an off center emboli capture filter 70, that is, a capture filter that deploys non-coaxially with respect to the axis of the shaft 10. The capture filter 70 effectively forces the shaft of the stent deployment system into an off center arrangement. In the embodiment of the figure, the capture filter 70 engages with guide wire 3, which extends distally, relative to distal member 20. Specifically, capture filter 70 is provided with support hoop 100' and blood permeable sac 102 affixed to support hoop 100'. Sac 102 is coupled to support hoop 100' so that the support hoop 24 forms an opening for the sac. In Figure 2, support hoop 100' is preferably connected to guide wire 3 near distal end 23 of the guide wire, yet relatively proximate to distal tip 20 of device 1. Also, as shown therein, the distal end of sac 102' is preferably anchored at bearing 104, though an arrangement where distal end of sac is unanchored is acceptable as well.

Sac 102 may be constructed of a thin, flexible biocompatible material, such as polyethylene, polypropylene, polyurethane, polyester, polyethylene terephthalate, nylon or polytetraflouroethylene, or combinations thereof. Sac 102 includes openings or pores 106 sized to permit blood cells to pass through the sac without restraint, while filtering larger emboli, thrombus, or foreign bodies that may be released during a medical procedure, such as angioplasty or stent placement. The openings or pores 106 in sac 102 have a diameter range of about 20 to 400 microns in diameter, and more preferably, about approximately 80 microns. These pore sizes permit red blood cells (which have a diameter of approximately 5 microns) to easily pass through the sac, while capturing thrombus or emboli.

Support hoop 100' preferably comprises nitinol wire, although it may also be formed from a multi-strand nitinol cable, a spring tempered stainless steel, or other super-elastic material. Support hoop 24 also may include radiopaque features, such as gold or platinum bands 33, spaced at intervals around the circumference of support hoop 24, or a coil of radiopaque material wrapped around the support hoop, or a gold plated coating.

The support hoop 100' and blood permeable sac 102 retained in a delivery state within the lumen of sheath 40 or distal tip 20 (Fig. 4. illustrates an arrangement where the distal tip 20 houses hoop 100' and sac 102). In use, guide wire 3 is manipulated into position proximal to stenosis within vessel using well-known percutaneous techniques. Stent 50 is disposed in its contracted delivery state within the distal end of sheath 40. The device is advanced through the vessel using guide wire 3. Support hoop 100' is compressed, for example, by folding the hoop in half.

With respect to Fig. 3, device 1 is disposed at the desired location proximal to stenosis within a patient's vessel, the distal end 110 of guide wire 3 is advanced through the lesion, until stent is positioned at the desired location. With the device in position, guide wire 3 is held stationary while sheath 40 is retracted. Alternatively, sheath 40 may be held stationary while guide wire 3 is advanced. In either case, when the filter 70 is no longer confined, support hoop 100' expands to seal against the walls of the vessel 5, expanding against the vessel walls and deploying blood permeable sac 102. Blood continues to flow through vessel 5. Furthermore, as the support hoop 100' of filter 70 is mounted to the guide wire 3 in a non-coaxial arrangement, it expands non-coaxially relative to the longitudinal axis of the shaft 10. The off center support member expands to and contacts the interior vessel wall, applying a counterforce to the interior shaft of the stent delivery system, forcing the stent delivery system into an off-center arrangement within the vessel. Thus, with the stent delivery system in the off center arrangement, and the physician continuing to retract the sheath, the self-expanding stent is exposed. In this off center arrangement in which the stent is deployed, it is believed that the stent is better anchored to the vessel walls, as the leading row of struts possessed by the stent are angled towards the vessel walls, as explained above.

In an alterative arrangement, sac 28 may be replaced with netting having the pore size that allows blood to flow while filtering out thrombosis. Merely by way of example, FilterWire, a product available from Boston Scientific, and Spider, by ev3 can be used as the material for constructing the filter.

In yet another embodiment, the off center support member can be retained within a sheath dedicated to the delivery and deployment of same. Such sheath can, for example, be positioned within the interior of the outer sheath 40, which retains the stent. In this arrangement, the delivery system is provided with the controls required to perform some or all of these functions: (1) move the dedicated sheath distally, prior to deployment off the off center support member, and (2) retract the sheath proximally, in order to deploy the off center support member, and (3) retract the sheath and OCRS within the outer sheath 40 after the stent is deployed in the off center arrangement. The operation and controls for operating stent delivery systems of the present kind to effect retraction and deployment of the delivery sheath and other components are well known in the art and a person of ordinary skill in the art would readily appreciate that such controls could be provided for operation of a lumen dedicated to the delivery of an off center support member. Merely by way of example, US-6,773,446 and US-6,939,352, disclose such controls.

## Claims

1. A delivery device (1) for delivering and deploying a medical device to a location in a patient's body requiring medical treatment, such as a blood vessel, comprising:
an inner shaft (10);
an outer sheath (40), which is coaxial with the inner shaft (10);
a self expanding stent (50) mounted on the inner shaft (10) and retained within the outer sheath (40); and
an operable portion comprising controls for moving the outer sheath (40) relative to the inner shaft (10); and **characterised in that** the delivery device further comprises:
an off center support member (100) in engagement with the inner shaft (10) and disposed interior the outer sheath (40) at a location distal to the stent (50), wherein said off center support member (100) is comprised of a self expanding material and engaged to the inner shaft (10) non coaxially, relative to the longitudinal axis of the inner shaft (10), and wherein the off center support member (100) is in an initial state of relative compression, restrained by a restraining force exerted by the outer sheath (40) and is able to expand when the restraining force is removed, such that when the device is in the patient's body, the off center support member (100), when expanded, is adapted to deploy against the vessel walls to displace the shaft (10) into an off center arrangement prior to the deployment of the stent (50).

2. A delivery device (1) for delivering and deploying a medical device to a location in a patient's body requiring medical treatment, such as a blood vessel, comprising:
an inner shaft (10) having a central lumen;
an outer sheath (40), which is coaxial with the inner shaft (10)
a self expanding stent (50) mounted on the inner shaft (10) and retained within the outer sheath (40); and
an operable portion comprising controls for moving the outer sheath (40) relative to the inner shaft (10); and **characterised in that** the delivery device further comprises:
an off center support member (100) in engagement with a guide wire (3) passing through the central lumen, the off center support member (100) being positioned at a location distal to the stent (50), wherein said off center support member (100) is comprised of a self expanding material and engaged to the guide wire (3) non coaxially, relative to the longitudinal axis of the inner shaft (10), wherein the off center support member (100) is in an initial state of relative compression, restrained by a restraining force exerted by the outer sheath (40) and is able to expand when the restraining force is removed, such that when the device is in the patient's body, the off center support member (100), when expanded, is adapted to deploy against the vessel walls to displace the shaft (10) into an off center arrangement prior to the deployment of the stent (50).

3. The device of claim 1 or 2, wherein the off center support member (100) is comprised of nitinol.

4. The device of claim 1 or 2, wherein the off center support member (100) is comprised of spring tempered stainless steel.

5. The device of claim 1 or 2, wherein the off center support member (100) is located distal to the stent (50) and proximal to a distal end of the device.

6. The device of claim 1 or 2, wherein the off center support member (100) is comprised of a vascular filter (70).

7. The device of claim 6, wherein the vascular filter (70) is comprised of an expandable hoop that forms the mouth of a blood permeable sac, which is engaged to the hoop.

8. The device of claim 1 further comprised of a guide wire (3) that extends through a central lumen of the inner shaft (10).

## Patentansprüche

1. Zuführungsvorrichtung (1) zum Zuführen und Entfalten einer medizinischen Vorrichtung an einen Ort in einem Körper eines Patienten, der einer medizinischen Behandlung bedarf, wie ein Blutgefäß, die Folgendes umfasst:
einen inneren Schaft (10);
eine äußere Hülse (40), die koaxial zu dem inneren Schaft (10) angeordnet ist;
einen selbstexpandierenden Stent (50), der auf dem inneren Schaft (10) montiert ist
und in der äußeren Hülse (40) zurückgehalten wird; und
einen Funktionsabschnitt, der Steuereinrichtungen zum Bewegen der äußeren Hülse (40) gegenüber dem inneren Schaft (10) umfasst; **dadurch gekennzeichnet, dass** die Zuführungsvorrichtung weiterhin Folgendes umfasst:
ein exzentrisches Trägerelement (100), das an dem inneren Schaft (10) angreift und innerhalb der äußeren Hülse (40) an einem Ort distal zu dem Stent (50) angeordnet ist, wobei das exzentrische Trägerelement (100) ein selbstexpandierendes Material umfasst und an dem inneren Schaft (10) nicht koaxial in Bezug auf die Längsachse des inneren Schafts (10) angreift und wobei das exzentrische Trägerelement (100) in einem Ausgangszustand vergleichsweise komprimiert ist, eingespannt durch eine Einspannkraft, die von der äußeren Hülse (40) aufgebracht wird, und in der Lage ist, sich zu expandieren, wenn die Einspannkraft entfernt wird, so dass, wenn sich die Vorrichtung in dem Körper des Patienten befindet, das exzentrische Trägerelement (100) dafür eingerichtet ist, sich beim Expandieren gegen die Gefäßwände zu entfalten, um den Schaft (10) in eine exzentrische Anordnung zu verlagern, bevor sich der Stent (50) entfaltet.

2. Zuführungsvorrichtung (1) zum Zuführen und Entfalten einer medizinischen Vorrichtung an einem Ort in einem Körper eines Patienten, der einer medizinischen Behandlung bedarf, wie ein Blutgefäß, die Folgendes aufweist:
einen inneren Schaft (10) mit einem zentrischen Lumen;
eine äußere Hülse (40), die koaxial zu dem inneren Schaft (10) angeordnet ist;
einen selbstexpandierenden Stent (50), der auf dem inneren Schaft (10) montiert ist und in der äußeren Hülse (40) zurückgehalten wird; und
einen Funktionsabschnitt, der eine Steuerung zum Bewegen der äußeren Hülse (40) gegenüber dem inneren Schaft (10) umfasst, **dadurch gekennzeichnet, dass** die Zuführungsvorrichtung weiterhin Folgendes umfasst:
ein exzentrisches Trägerelement (100), welches an einem Führungsdraht (3) angreift, der durch das zentrische Lumen führt, wobei das exzentrische Trägerelement (100) an einem Ort distal des Stents (50) angeordnet ist, wobei das exzentrische Trägerelement (100) ein selbstexpandierendes Material umfasst und an dem Führungsdraht (3) nicht koaxial in Bezug auf der Längsachse des inneren Schafts (10) angreift, wobei das exzentrische Trägerelement (100) in einem Ausgangszustand vergleichsweise komprimiert ist, eingespannt durch eine Einspannkraft, die von der äußere Hülse (40) aufgebracht wird, und in der Lage ist, sich zu expandieren, wenn die Einspannkraft entfernt wird, so dass, wenn sich die Vorrichtung in dem Körper des Patienten befindet, das exzentrische Trägerelement (100), dafür eingerichtet ist, sich beim Expandieren gegen die Gefäßwände zu entfalten, um den Schaft (10) in eine exzentrische Anordnung zu verlagern, bevor sich der Stent (50) entfaltet.

3. Vorrichtung nach Anspruch 1 oder 2, bei der das exzentrische Trägerelement (100) Nitinol umfasst.

4. Vorrichtung nach Anspruch 1 oder 2, bei der das exzentrische Trägerelement (100) wärmebehandelten Feder-Edelstahl umfasst.

5. Vorrichtung nach Anspruch 1 oder 2, bei der das exzentrische Trägerelement (100) distal zu dem Stent (50) und proximal zu dem distalen Ende der Vorrichtung angeordnet ist.

6. Vorrichtung nach Anspruch 1 oder 2, bei der das exzentrische Trägerelement (100) ein vaskuläres Filter (70) umfasst.

7. Vorrichtung nach Anspruch 6, bei welcher das vaskuläre Filter (70) ein expansionsfähigen Reifen umfasst, der die Öffnung eines blutdurchlässigen Beutels bildet, der an dem Reifen angreift.

8. Vorrichtung nach Anspruch 1, die weiterhin einen Führungsdraht (3) umfasst, der sich durch ein zentrales Lumen des inneren Schafts (10) erstreckt.

## Revendications

1. Dispositif de mise en place (1) pour mettre en place et déployer un dispositif médical sur un emplacement dans le corps d'un patient nécessitant un traitement médical, tel qu'un vaisseau sanguin, comprenant :
un arbre interne (10) ;
une gaine externe (40), qui est coaxiale par rapport à l'arbre interne (10) ;
une endoprothèse vasculaire auto-étendue (50) montée sur l'arbre interne (10) et retenue dans la gaine externe (40) ; et
une partie actionnable comprenant des commandes pour déplacer la gaine externe (40) par rapport à l'arbre interne (10) ; et **caractérisé en ce que** le dispositif de mise en place comprend en outre :
un élément de support excentré (100) en prise avec l'arbre interne (10) et disposé à l'intérieur de la gaine externe (40) à un emplacement distal par rapport à l'endoprothèse vasculaire (50), où ledit élément de support excentré (100) est constitué d'un matériau auto-étendu et en prise avec l'arbre interne (10) de manière non coaxiale, par rapport à l'axe longitudinal de l'arbre interne (10), et où l'élément de support excentré (100) est dans un état initial de compression relative, retenu par une force de retenue exercée par la gaine externe (40) et capable de s'étendre lorsque la force de retenue est retirée, de sorte que lorsque le dispositif est dans le corps du patient, l'élément de support excentré (100), une fois étendu, est adapté pour se déployer contre les parois de vaisseau afin de déplacer l'arbre (10) dans un agencement excentré avant le déploiement de l'endoprothèse vasculaire (50).

2. Dispositif de mise en place (1) pour mettre en place et déployer un dispositif médical sur un emplacement dans le corps d'un patient nécessitant un traitement médical, tel qu'un vaisseau sanguin, comprenant :
un arbre interne (10) ayant une lumière centrale ;
une gaine externe (40), qui est coaxiale par rapport à l'arbre interne (10) ;
une endoprothèse vasculaire auto-étendue (50) montée sur l'arbre interne (10) et retenue dans la gaine externe (40) ; et
une partie actionnable comprenant des commandes pour déplacer la gaine externe (40) par rapport à l'arbre interne (10) ; et **caractérisé en ce que** le dispositif de mise en place comprend en outre :
un élément de support excentré (100) en prise avec un fil guide (3) traversant la lumière centrale, l'élément de support excentré (100) étant positionné à un emplacement distal par rapport à l'endoprothèse vasculaire (50), où ledit élément de support excentré (100) est constitué d'un matériau auto-étendu et en prise avec le fil guide (3) de manière non coaxiale, par rapport à l'axe longitudinal de l'arbre interne (10), où l'élément de support excentré (100) est dans un état initial de compression relative, retenu par une force de retenue exercée par la gaine externe (40) et également capable de s'étendre lorsque la force de retenue est retirée, de sorte que lorsque le dispositif est dans le corps du patient, l'élément de support excentré (100), une fois étendu, est adapté pour se déployer contre les parois de vaisseau afin de déplacer l'arbre (10) dans un agencement excentré avant le déploiement de l'endoprothèse vasculaire (50).

3. Dispositif selon la revendication 1 ou 2, dans lequel l'élément de support excentré (100) est constitué de nitinol.

4. Dispositif selon la revendication 1 ou 2, dans lequel l'élément de support excentré (100) est constitué d'acier inoxydable à ressort.

5. Dispositif selon la revendication 1 ou 2, dans lequel l'élément de support excentré (100) est situé de manière distale par rapport à l'endoprothèse vasculaire (50) et de manière proximale par rapport à une extrémité distale du dispositif.

6. Dispositif selon la revendication 1 ou 2, dans lequel l'élément de support excentré (100) est constitué d'un filtre vasculaire (70).

7. Dispositif selon la revendication 6, dans lequel le filtre vasculaire (70) est constitué d'un cercle extensible qui forme l'ouverture d'un sac perméable au sang, qui est en prise avec le cercle.

8. Dispositif selon la revendication 1, constitué en outre d'un fil guide (3) qui s'étend à travers une lumière centrale de l'arbre interne (10).
